# EUROPEAN PATENT APPLICATION

(11) **EP 4 005 642 A1**
(43) Date of publication of application: **01.06.2022**
(21) Application number: 20928473.6
(22) Date of filing: 08.09.2020
(51) Int. Cl.: A62B 7/00, A62B 18/02, A41D 13/11

(54) **RESPIRATORY SYSTEM PROTECTION DEVICE**

(30) Priority: 30.03.2020 RU 2020112706
(71) Applicant: Zege, Sergey Olegovich, Moscow, 125319 (RU); Zege, Oleg Sergeevich, Moscow, 125319 (RU)
(72) Inventor: Zege, Sergey Olegovich, Moscow, 125319 (RU); Zege, Oleg Sergeevich, Moscow, 125319 (RU)
(74) Representative: Jeck, Jonathan
(86) International application number: PCT/RU2020/000472
(87) International publication number: WO 2021/201716

(57) **Abstract**

A respiratory system protection device comprises a helmet, which is hermetically connected to a mask, and also an apparatus for cleaning outgoing air, which apparatus is connected to the outlet of a discharge tube, wherein a valve for controlling the pressure of incoming air is installed at the inlet of a supply tube, and an apparatus for cleaning incoming air comprises a pump which, by means of the supply tube, feeds incoming air into the helmet under a pressure which is higher than atmospheric pressure, wherein the apparatus for cleaning the internal air is capable of cleaning the internal air of aggressive and biological substances.

## Description

### Area of technology

The proposed invention relates to devices for the protection of the respiratory organs against harmful substances. Such inventions include gas masks. The gas mask includes a helmet. Gas masks can be used in industrial production. They can also be used in natural and man-made situations. Personal protection is required in such situations. The device protects against noxious gases, particulates and biological agents. The latter is spread by airborne droplets.

The main distinguishing feature of the present invention is the creation of a respiratory protection device. Such a device will ensure that the incoming air stream is cleaned as from aggressive chemical and biological substances. The device will provide protection of the air exiting this device and from biological agents. Protection is also ensured by using the device to clean the outgoing air stream. Also, the device will provide a high degree of tightness due to the use of a helmet with a mask. The device purifies the incoming air. Also it provides an inlet air purification device with increased pressure inside the mask with respect to the ambient pressure which provides an increase in the purity of air for breathing.

### Prior art

There are products that trap dust and biological particles. These products are respirators (e.g. Alina-310 half mask or SPIROTEX 2200 CV respirator). Respirators do not provide sufficient protection. The wearer's respiratory system is inadequately protected against external influences. This is because inhalation and exhalation take place through the respirator in the same direction.

The closest to the claimed invention is a "Filtering gas mask". The gas mask is described in the RF utility model patent N°137205, A62B 18/00. The gas mask contains a sealed mask. It contains an exhalation valve unit and an inhalation valve unit. The valve assembly is connected to a filter-absorption box and tied together using a connecting tube. A negative ion source module is incorporated into the exhalation valve assembly. This gas mask is selected as a prototype of the claimed invention.

The disadvantage of the known gas mask is the insufficient degree of protection. Protection from exhaled air of surrounding persons. Protection against aggressive biological substances. These substances are present in the human body. The person using the gas mask.

The design of the known gas mask also does not provide adequate protection against penetration. The penetration of external, untreated air into the mask.

Also the known gas mask has an inconvenience. Uncomfortable to wear. Due to the fit of the mask against the user's skin. Also has an unattractive appearance.

### The essence of the invention

The purpose of this invention is to create a device for respiratory protection. The device will purify the incoming air stream from aggressive chemical and biological substances. It will also provide protection of the air exiting this device e.g protection against biological substances. Protection by using a device to clean the exhaust air. Also, the invention will provide a high degree of tightness of this device. The degree of tightness will be provided by a helmet with a mask. Protection will be provided by the use of a high-pressure incoming air cleaner.

The objective is achieved by creating a respiratory protection device. The device contains a mask. The mask is connected to the outlet of an inlet tube. The inlet of the tube is connected to an inlet air cleaner. The inlet of the discharge tube is connected to the mask. The device contains a helmet. The helmet is hermetically connected to the mask.

The device contains an air outlet cleaning device. The device is connected to the outlet of the extraction tube. There is a valve at the inlet of the supply pipe to adjust the inlet air pressure. The inlet air cleaner contains a pump. The pump supplies the helmet with pressurised incoming air through the supply pipe. The pressure is higher than atmospheric pressure. The inlet air purifier can purify the internal air from aggressive and biological substances. And it can discharge the purified air to the outside. And the inlet air cleaner can draw in outside air through the inlet. The unit can clean the outside air from aggressive chemical and biological substances. The unit can create a pressure of outside air that is higher than atmospheric pressure. The air is routed into the inlet tube and the helmet and mask.

In the preferred embodiment of the device being created, the helmet with mask contains a flexible membrane. The membrane will ensure that the helmet adheres tightly to the user's body.

The device should preferably have tubes for ventilation. One of the tubes is preferably fitted to the front of the helmet. The other is preferably fitted to the back of the helmet. The front (first) ventilation tube has a rigid section at the front of the helmet. Also has a flexible section. The rear (second) ventilation tube has a rigid section at the back of the helmet. It also has a flexible section.

It is advisable for the device to have a rigid section of the front ventilation tube with openings for air to enter the helmet. The rigid section of the rear ventilation tube has holes for venting air from the helmet.

Preferably, the inlet air cleaner in the appliance is positioned at the top of the appliance. At the top of the user's left and right hand. The inlet air cleaning device is preferably placed as well on the top of the user's left and right hand.

It is advisable for the unit to contain quartz lamps which are mounted on the outside of the unit.

Preferably, the unit should contain an operation monitoring unit. A monitoring unit with a means of signalling a malfunction of the unit.

Preferably, in the device, the lead-in tube is located on the front side of the helmet. The diverter tube is located on the rear side of the helmet.

It is advisable for the unit to have a battery-operated, electrically driven incoming air cleaner pump and/or a mechanical drive.

### BRIEF DESCRIPTION OF THE DRAWINGS

For a more detailed description of the invention, a description of specific possible embodiments thereof with the corresponding drawings is now described.
Figure 1 shows a front view of an apparatus for protecting respiratory organs made according to the invention.
Figure 2 shows a rear view of an apparatus for protecting respiratory organs made according to the invention.

### Best embodiments of the invention are described

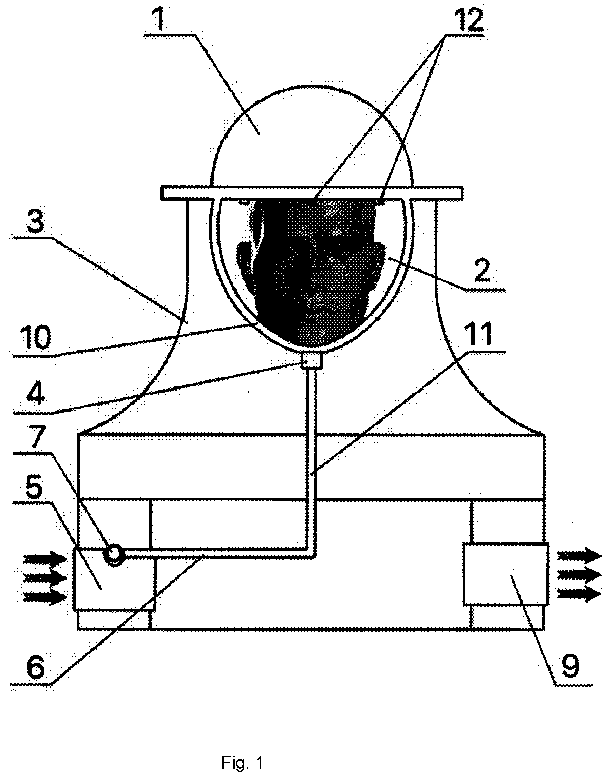

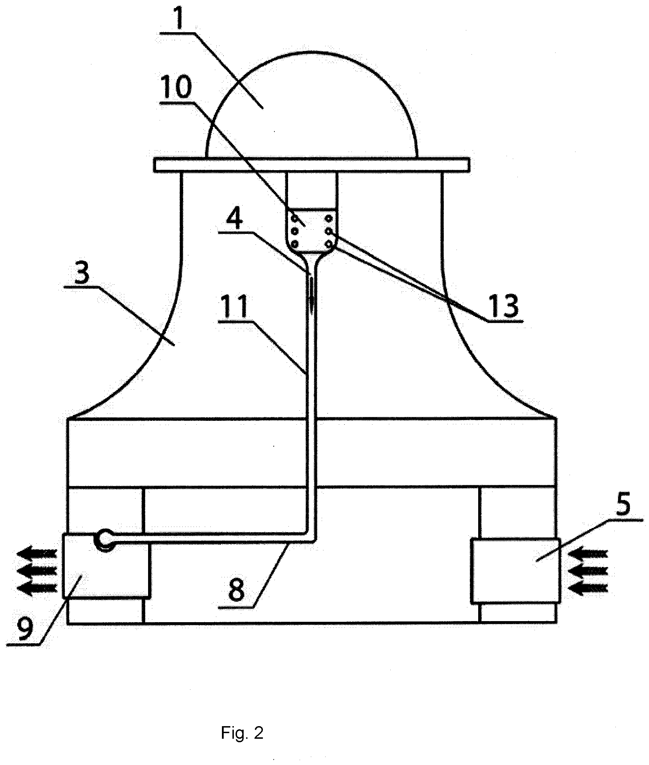

In a preferred embodiment of the invention, the respiratory protection device comprises a helmet 1 comprising a transparent mask 2 and a flexible membrane 3, an air ventilation tube 4, an incoming air cleaning device 5, a supply tube 6, a pressure regulating valve 7, a discharge tube 8, an outlet air cleaning device 9, a rigid section 10 of the air ventilation pipe 4, a flexible section 11 of the air ventilation pipe 4, an air inlet hole 12 in the helmet 1, and an air discharge opening 13 (Fig. 1, 2).

The device for protecting the respiratory organs functions as follows.

Ambient air is supplied to helmet 1 with mask 2 from incoming air cleaning device 5 located on helmet or user's body, which filters external air, increases its pressure, and under increased pressure supplies air through valve for regulation of pressure 7 into supply tube 6. Then air gets into flexible section 11 of front tube for ventilation 4, and then enters its rigid section 10, after which air enters helmet 1 through introducing air 12. Inside helmet 1 external air is inhaled by the user. The user exhales through holes for venting air 13 in rigid section 10 of rear tube for ventilation 4. The air enters flexible section 11 of this tube for ventilation 4 and then enters discharge tube 8, from which it is supplied to device for cleaning of outgoing air 9, located on helmet or body of user, which cleans internal air from aggressive biological substances exhaled by userand outputs the purified air to the outside (FIG. 1, 2).

Also, the device for protecting the respiratory organs can have a unit for monitoring the operation of the device (not shown in the figures) with a device for indicating the malfunction of the device, which, in the event of a deviation of the operating modes of the device, comprises signalling the device malfunction.

In this way, the user's breathing is carried out with air passed through the external air cleaning device 5 with the required level of cleaning. At the same time, due to excessive pressure inside the helmet 1, air is excluded from outside, bypassing the device for cleaning incoming air, which significantly increases the tightness of the claimed device.

The presence of the device for purifying outgoing air 9 minimizes the presence of aggressive chemical and biological substances exhaled by the user in the air, which has passed through the claimed device, which significantly expands the functionality of the claimed device, makes it possible not only to clean incoming air, but also to clean from biological substances and output air from the device air.

The presence of a pump with an electric or mechanical drive for the operation of the incoming air cleaning device 5 makes it possible to reduce the load on the user's respiratory system, wherein the load does not exceed the load during normal breathing of the user without the use of the claimed device.

Use of helmet 1 and mask 2 around head excludes touching of mucous membranes and skin of user's face with possible contaminated internal surface of helmet 1 and mask 2, and flexible membrane 3 provides tight adjacency of helmet 1 to user's body.

Possible presence in the claimed device of quartz lamps provides additional disinfection of air outside the device. In this case, the specific mark of the quartz lamp is selected according to the conditions of the environment in which this device is to be used for as well as to the degree of cleaning.

The use of the claimed device by a several people at the same time in the same place makes it possible to significantly reduce the possibility of transmitting chemical and biological substances by an air-droplet method, which allows members of such groups to interact with each other, excluding the possibility of mutual contamination.

The use of the claimed device by the individual user makes it possible to protect its respiratory organs from external action and to exclude the transfer of chemical and biological substances inherent thereto by an air-droplet method.

The design of the claimed device for protecting respiratory organs makes it attractive for wearing in everyday life, in transport and in entertainment events.

The distinctive feature of the claimed device for respiratory protection is the presence of a ventilation system which is provided by tubes for ventilation 4, and provides a user with clean external air and purifies the air exhaled by the user, wherein the most massive parts of the device are placed on the user's body, and the mask 2 does not come in contact with the skin of the face.

As will be appreciated by those skilled in the art, the present invention is easy to design in other specific forms without departing from the essence of the present invention.

The present embodiments are merely illustrative and not restrictive, the scope of the invention being represented by the claims, and it is intended that all possible changes and areas of equivalency of the claims are intended to be included therein.

## Claims

1. Respiratory protective device comprising:
- a mask (2),
- an outlet of an inlet tube (6) is connected to the mask,
- the inlet tube is connected to an inlet air cleaner (5), which contains a diverting tube (8),
- an inlet of a discharge tube is connected to the mask (2),
**characterized in that**
- it comprises a helmet (1),
- the helmet is hermetically connected to the mask (2), which comprises an exhaust air cleaning device (9),
- the device is connected to the outlet of the discharge tube (8)
- a valve is provided at the inlet of the supply tube (6),
- a valve is provided for adjusting the pressure (7) of the incoming air and
- the inlet air cleaner (5) contains a pump, which supplies the helmet (1) with pressurised incoming air,
- the pressure inside the mask is provided higher than atmospheric pressure,
- the pump supplies the incoming air through the supply tube (6),
- the exhaust air cleaning device (9) is adapted to clean the internal air from aggressive and biological substances, for discharge of purified air to the outside,
- the inlet air cleaner (5) is adapted to take in outside air through the inlet, is adapted to clean the outside air from aggressive chemical and biological substances, and is adapted generate an air pressure in the mask that is increased relative to atmospheric pressure, directed into the inlet tube (6) and the helmet (1) with the mask (2).

2. Device according to claim 1,
**characterized in that**
the helmet (1) with mask (2) contains a flexible membrane (3), which membrane enables the helmet (1) to fit tightly onto the user's body.

3. Device according to claim 1,
**characterized in that**
- it has tubes for air ventilation (4), wherein one of the tubes is fitted to the front of the helmet (1), another tube is fitted to the back of the helmet (1),
- the front air ventilation tube has a rigid section (10), which is mounted in the front of the helmet (1), also the front air ventilation tube also has a flexible section (11), which is connected to the supply tube (6), and/or
- the rear ventilation tube (4) has a rigid section (10), which is mounted in the rear part of the helmet (1), which has a flexible section (11) that is connected to the diverter tube (6).

4. Device according to claim 3,
**characterized in that**
the rigid section (10) of the front venting tube (4) has holes for introducing air (12) into the helmet (1) and the rigid section (10) of the rear venting tube (4) has holes for venting air (13) from the helmet (1).

5. Device according to claims 1,
**characterized in that**
the inlet air cleaning device (5) and the outlet air cleaning device (9) are placed in the upper part of the user's left and right hand respectively.

6. Device according to claim 1,
**characterized in that**
it comprises quartz lamps, which are mounted on the outside of the appliance.

7. Device according to claim 1,
**characterized in that**
it comprises a device operation monitoring unit with a device fault signalling facility.

8. Device according to claim 1,
**characterized in that**
the incoming air cleaner pump is electrically driven with a battery and/or has a mechanical drive.
